# EUROPEAN PATENT APPLICATION

(11) **EP 2 042 481 A1**
(43) Date of publication of application: **01.04.2009**
(21) Application number: 07743107.0
(22) Date of filing: 10.05.2007
(51) Int. Cl.: C07C 211/58, C09K 11/06, H01L 51/50

(54) **AROMATIC AMINE DERIVATIVE, AND ORGANIC ELECTROLUMINESCENCE DEVICE USING THE SAME**

(30) Priority: 27.06.2006 JP 2006177029
(71) Applicant: Idemitsu Kosan Co., Ltd., Chiyoda-ku Tokyo 100-8321 (JP)
(72) Inventor: YABUNOUCHI, Nobuhiro, Sodegaura-shi, Chiba 299-0293 (JP); KAWAMURA, Masahiro, Sodegaura-shi, Chiba 299-0293 (JP)
(74) Representative: Gille Hrabal Struck Neidlein Prop Roos
(86) International application number: PCT/JP2007/059672
(87) International publication number: WO 2008/001551

(57) **Abstract**

A novel aromatic amine derivative which is a diamine compound having a p-terphenyl-4,4"-diyl structure. An organic electroluminescence device which is composed of one or more organic thin film layers including at least one light emitting layer sandwiched between a cathode and an anode. Since at least one of the organic thin film layers contains the aromatic amine derivative singly or combination of two or more, the tendency of molecular crystallization is reduced and the yield in the fabrication of electroluminescence devices is enhanced. The aromatic amine derivative enables the production of electroluminescence devices having a prolonged lifetime and a high efficiency even after storing at high temperatures.

## Description

### TECHNICAL FIELD

The present invention relates to an aromatic amine derivative and an organic electroluminescence (EL) device using the same. More particularly, it relates to an organic EL device with reduced tendency of molecular crystallization, with an enhanced yield in fabricating it and with long lifetime, and an aromatic amine derivative realizing the device.

### BACKGROUND ART

An organic EL device is a spontaneous light emitting device which utilizes the phenomenon that a fluorescent substance emits light by energy of recombination of holes injected from an anode and electrons injected from a cathode when an electric field is applied. Since an organic EL device of the laminate type driven under a low electric voltage was reported by C. W. Tang et al. of Eastman Kodak Company (C. W. Tang and S. A. Vanslyke, Applied Physics Letters, Volume 51, Page 913, 1987), many studies have been conducted on organic EL devices using organic materials as the constituting materials. Tang et al. used tris(8-quinalinolato)aluminum for the light emitting layer and a triphenyldiamine derivative for the hole transporting layer. Advantages of the laminate structure are that the efficiency of hole injection into the light emitting layer can be increased, that the efficiency of forming excitons which are formed by blocking and recombining electrons injected from the cathode can be increased, and that the excitons formed in the light emitting layer can be confined. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

Usually, driving or storing the organic EL devices under an environment of elevated temperatures causes adverse influences such as changes of luminescent colors, degradation of current efficiency of light emission, increase of their driving voltage, reduction of lifetime in their light emission, etc. In order for preventing the adverse influences, it was necessary to elevate a glass transition temperature (Tg) of a hole transporting material. Accordingly, it is necessary for the hole transporting materials to have many aromatic groups in their molecules (for example, aromatic diamine derivatives disclosed in Patent Document 1, aromatic fused ring diamine derivatives disclosed in Patent Document 2), and usually, structures having 8 to 12 benzene rings are preferably employed.
However, when the hole transporting materials have many aromatic groups in their molecules, the thin-film formation for fabricating the organic EL device employing them tends to cause crystallization, resulting in problems such as blocking of the opening of crucibles used in vapor deposition, defects in the thin film caused by crystallization, and the reduction in the yield of the organic EL devices. Further, although the glass transition temperature (Tg) is generally high, compounds having many aromatic groups in their molecules have elevated sublimation temperatures and short lifetimes because of possible phenomena such as non-uniform decompositions during vapor depositions and non-uniform depositions.
Some documents disclose asymmetric aromatic amine derivatives. For example, Patent Document 3 discloses the aromatic amine derivatives having asymmetric structure, however, it describes neither specific examples nor properties of the asymmetric compounds. Moreover, although Patent Document 4 discloses asymmetric aromatic amine derivatives having phenanthrene in its working example, the document recognizes the asymmetric compounds equivalent to symmetric compounds with respect to the properties as EL materials and describes nothing about the properties of the asymmetric compounds. The asymmetric compounds are produced by special synthetic processes. However, the above patent documents do not clearly describe the production methods of the asymmetric compounds. Further, although Patent Document 5 discloses a production method of the aromatic amine derivatives having asymmetric structure, the document fails to describe the properties of the asymmetric compounds. Patent Document 6 describes thermally stable asymmetric compounds having high glass transition temperatures, however, only a compound having a carbazole structure is exemplified.

Further, some documents disclose aromatic amine derivatives having a p-terphenyl-4,4"-diyl structure (Patent Documents 7 to 13). However, the compound described in Patent Document 7 is not preferable because defects are caused in thin film by crystallization. Patent Documents 8 and 9 only describe the structures of the compounds having a p-terphenyl-4,4"-diyl structure without describing their properties. The scope of the claims of Patent Document 10 includes the p-terphenyl-4,4"-diyl derivative, however, the typical examples and working examples include no p-terphenyl-4,4"-diyl derivative. Further, Patent Document 10 is different from the present invention in that the compounds of Patent Document 10 contain a heteroring. Although Patent Document 11 discloses a p-terphenyl-4,4"-diyl derivative having a t-butyl group, the proposed compound is not suitable as a hole transporting material because of its short lifetime. The scope of the claims of Patent Document 12 includes a p-terphsnyl-4,4"-diyl derivative, however, the typical examples and working examples include no p-terphenyl-4,4"-diyl derivative. Although Patent Document 13 discloses a p-terphenyl-4,4"-diyl derivative, it fails to teach a compound having a terminal p-terphenyl group. Patent Document 14 describes a p-terphenyl-4,4"-diyl derivative and a compound having a terminal fused ring, however, effects of the compound are not described.
As mentioned above, there are many reports about the organic EL devices with prolonged lifetime. However, the lifetime is still insufficient. Accordingly, the development of an organic EL device having superior performance has been eagerly desired.

Patent Document 1: U.S. Pat. No. 4,720,432
Patent Document 2: U.S. Pat. No. 5,061,569
Patent Document 3: JP 8-48656A
Patent Document 4: JP 11-135261A
Patent Document 5: JP 2003-171366A
Patent Document 6: U.S. Pat. No. 6,242,115
Patent Document 7: Japanese Patent No. 3398548
Patent Document 8: JP 11-149986A
Patent Document 9: JP 11-312587A
Patent Document 10: JP-T-2004-536134
Patent Document 11: JP 11-167991A
Patent Document 12: JP 2002-53533A
Patent Document 13: Japanese Patent Application No. 2003-007762
Patent Document 14: Japanese Patent Application No. 2004-206969

### DISCLOSURE OF THE INVENTION

### PROBLEMS TO BE SOLVED BY THE INVENTION

The present invention has been made to overcome the above problems and has an object of providing an organic EL device with reduced tendency of molecular crystallization, with an enhanced yield in fabricating it, with long lifetime and further, having an enhanced efficiency of light emission; and an object of providing an aromatic amine derivative realizing the EL device.

### MEANS FOR SOLVING THE PROBLEM

As a result of intensive researches and studies to achieve the above object, the inventors have found that the above problems are overcome by using a novel aromatic amine derivative represented by the following general formula (1), which has a specified substituent, as a material for the organic EL device, especially as a hole transporting material, thereby completing the present invention.
Further, it has been found that a novel aromatic diamine derivative having a p-terphenyl-4,4"-diyl structure shows a small intermolecular interaction because of its steric hindrance, thereby preventing the crystallization, improving the yield in fabricating organic EL devices, prolonging the lifetime, and enhancing the efficiency of light emission. Especially, it has been found that remarkable effects of the enhanced efficiency of light emission are obtainable by combining the derivative with an EL device emitting blue light.

Namely, the present invention provides an aromatic amine derivative represented by the following general formula (1): 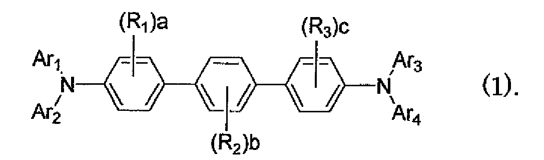 In the general formula (1), Ar₁ to Ar₄ each independently represents a substituted or unsubstituted aryl group having 5 to 50 ring atoms and at least one of Ar₁ to Ar₄ represents a substituted or unsubstituted aromatic fused ring group having 5 to 50 ring atoms;
R₁ to R₃ each independently represents a hydrogen atom, a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxy group or a carboxy group; and
a, b and c each independently represents an integer of 0 to 4;
with the proviso that when any of a, b and c is an integer of 2 or greater, plural groups of corresponding Ri to R₃ may be bonded to each other to form a saturated or unsaturated, five- or six-membered ring structure which may be substituted.

Further, the present invention provides an organic EL device which is composed of one or more organic thin film layers including at least one light emitting layer and sandwiched between a cathode and an anode, wherein at least one of the organic thin film layers contains the aromatic amine derivative singly or in combination of two or more.

### EFFECT OF THE INVENTION

The aromatic amine derivative and the organic EL device employing the aromatic amine derivative of the present invention shows a reduced tendency for molecular crystallization, with an enhanced yield in fabricating it, with a long lifetime and further, having an enhanced efficiency of light emission.

### PREFERRED EMBODIMENT TO CARRY OUT THE INVENTION

The present invention provides an aromatic amine derivative represented by a following general formula (1): 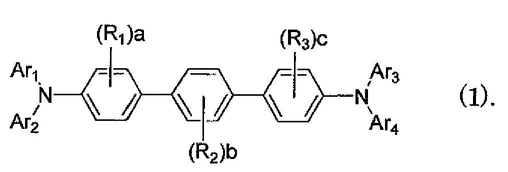 In the general formula (1), Ar₁ to Ar₄ each independently represents a substituted or unsubstituted aryl group having 5 to 50 ring atoms and at least one of Ar₁ to Ar₄ represents a substituted or unsubstituted aromatic fused ring group having 5 to 50 ring atoms; and
R₁ to R₃ each independently represents a hydrogen atom, a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxy group or a carboxy group.

Examples of the substituted or unsubstituted aryl group having 5 to 50 ring atoms represented by Ar₁ to Ar₄ and R₁ to R₃ in the general formula (1) include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl) phenyl group, 3-methyl-2-naphthyl group, 4-methyl-1-naphthyl group, 4-methyl-1-anthryl group, 4'-methylbiphenylyl group, 4"-t-butyl-p-terphenyl 4-yl group, fluoranthenyl group, fluorenyl group, 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthroline-2-yl group, 1,7-phenanthroline-3-yl group, 1,7-phenanthroline-4-yl group, 1,7-phehanthroline-5-yl group, 1,7-phenanthrohne-6-yl group, 1,7-phenanthroline-8-yl group, 1,7-phenanthraline-9-yl group, 1,7-phenanthroline-10-yl group, 1,8-phenanthroline-2-yl group, 1,8-phenanthroline-3-yl group, 1,8-phenanthroline-4-yl group, 1,8-phenanthroline-5-yl group, 1,8-phenanthroline-6-yl group, 1,8-phenanthrohne-7-yl group, 1,8-phenanthroline-9-yl group, 1,8-phenanthroline-10-yl group, 1,9-phenanthroline-2-yl group, 1,9-phenanthroline-3-yl group, 1,9-phenanthroline-4-yl group, 1,9-phenanthroline-5-yl group, 1,9-phenanthroline-6-yl group, 1,9-phenanthroline-7-yl group, 1,9-phenanthroline-8-yl group, 1,9-phenanthroline-10-yl group, 1,10-phenanthroline-2-yl group, 1,10-phenanthroline-3-yl group, 1,10-phenanthroline-4-yl group, 1,10-phenanthroline-5-yl group, 2,9-phenanthroline-1-yl group, 2,9-phenanthroline-3-yl group, 2,9-phenanthroline-4-yl group, 2,9-phenanthroline-5-yl group, 2,9- phenanthroline-6-yl group, 2,9-phenanthroline-7-yl group, 2,9-phenanthroline-8-yl group, 2,9-phenanthroline-10-yl group, 2,8-phenanthroline-1-yl group, 2,8-phenanthroline-3-yl group, 2,8-phenanthroline-4-yl group, 2,8-phenanthroline-5-yl group, 2,8-phenanthroline-6-yl group, 2,8-phenanthroline-7-yl group, 2,8-phenanthroline-9-yl group, 2,8-phenanthroline-10-yl group, 2,7-phenanthroline-1-yl group, 2,7-phenanthroline-3-yl group, 2,7-phenanthroline-4-yl group, 2,7-phenanthroline-5-yl group, 2,7-phenanthroline-6-yl group, 2,7-phenanthroline-8-yl group, 2,7-phenanthroline-9-yl group, 2,7-phenanthroline-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrole-1-yl group, 2-methylpyrrole-3-yl group, 2-methylpyrrole-4-yl group, 2-methylpyrrole-5-yl group, 3-methylpyrrole-1-yl group, 3-methylpyrrole-2-yl group, 3-methylpyrrole-4-yl group, 3-methylpyrrole-5-yl group, 2-t-butylpyrrole-4-yl group, 3-(2-phenylpropyl)pyrrole-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, etc.
Among those, phenyl group, naphthyl group, biphenylyl group, anthryl group, terphenylyl group, phenanthryl group, pyrenyl group, crycenyl group, fluoranthenyl group and fluorenyl group are preferable.

Examples of the substituted or unsubstituted alkyl group having 1 to 50 carbon atoms represented by R₁ to R₃ in the general formula (1) include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitro isobutyl group, 1,2-dinitro ethyl group, 1,3-dinitroisopropyl group, 2,3-dinitro-t-butyl group, 1,2,3-tranitropropyl group, cyclopropyl group, cyclobutyl group, cyclopentyl group, cyclohexyl group, 4-methylcyclohexyl group, 1-adamantyl group, 2-adamantyl group, 1-norbornyl group, 2-norbornyl group, etc.

The substituted or unsubstituted alkoxy group having 1 to 50 carbon atoms represented by R₁ to R₃ in the general formula (1) is a group expressed by -OY and examples of Y are the same as described about the foregoing alkyl group.
Examples of the substituted or unsubstituted aralkyl group having 6 to 50 carbon atoms represented by R₁ to R₃ in the general formula (1) include benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphtbylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group,o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, 1-chloro-2-phenyhsopropyl group, etc.

The substituted or unsubstituted aryloxy group having 5 to 50 ring atoms represented by R₁ to R₃ in the general formula (1) is a group expressed by -OY' and examples of Y' are the same as described about the foregoing aryl group.
The substituted or unsubstituted arylthio group having 5 to 50 ring atoms represented by R₁ to R₃ in the general formula (1) is a group expressed by -SY' and examples of Y' are the same as described about the foregoing aryl group.
The substituted or unsubstituted alkoxycarbonyl group having 2 to 50 carbon atoms represented by R₁ to R₃ in the general formula (1) is a group expressed by -COOY and examples of Y are the same as described about the foregoing alkyl group.
Examples of the aryl group in the amino group substituted by the aryl group having 5 to 50 ring atoms represented by R₁ to R₃ in the general formula (1) are the same as described about the foregoing aryl group.
Examples of the halogen atom represented by R₁ to R₃ in the general formula (1) include fluorine atom, chlorine atom, bromine atom, and iodine atom.

In the general formula (1), a, b and c each independently represents an integer of 0 to 4. When any of a, b and c is an integer of 2 or greater, plural groups of R₁ to R₃ may be bonded to each other to form a saturated or unsaturated, five- or six-membered ring structure which may be substituted.
Examples of the five- or six-membered ring structure include a cycloalkane having 4 to 12 carbon atoms such as cyclopentane, cyclohexane, adamantane, norbornane, etc.; a cycloalkene having 4 to 12 carbon atoms such as cyclopentene, cyclohexene, etc.; a cycloalkadiene having 6 to 12 carbon atoms such as cyclopentadiene, cyclohexadiene, etc.; an aromatic ring having 6 to 50 carbon atoms such as benzene, naphthalene, phenanthrene, anthracene, pyrene, chrysene, acenaphthylene, etc.

It is preferable for the aromatic amine derivative of the present invention that both Ar₁ and Ar₃ in the general formula (1) are substituted or unsubstituted aromatic fused ring groups having 5 to 50 ring atoms.
It is preferable for the aromatic amine derivative of the present invention that both Ar₁ and Ar₃ in the general formula (1) are α-naphthyl groups or β-naphthyl groups.
It is preferable for the aromatic amine derivative of the present invention that Ar₂ and Ar₄ in the general formula (1) are the same.
It is preferable for the aromatic amine derivative of the present invention that both Ar₂ and Ar₄ in the general formula (1) are phenyl groups.
It is preferable for the aromatic amine derivative of the present invention that both Ar₂ and Ar₄ in the general formula (1) are biphenylyl groups.
It is preferable for the aromatic amine derivative of the present invention that both Ar₂ and Ar₄ in the general formula (1) are terphenylyl groups.

The aromatic amine derivatives of the present invention are preferably used as the materials for the organic EL devices.
The aromatic amine derivatives of the present invention are preferably used as the hole transporting materials for the organic EL devices.

Specific examples of the aromatic amine derivative represented by the general formula (1) of the present invention are shown below, though not particularly limited thereto.

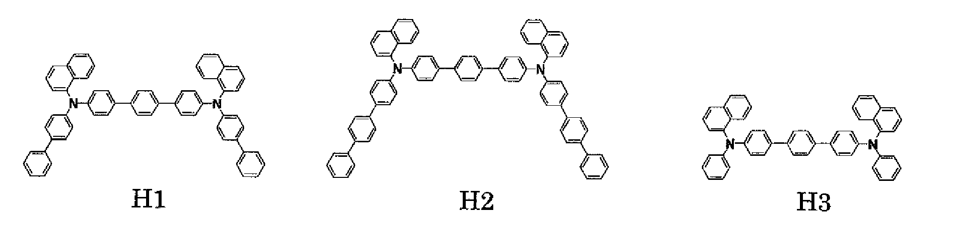

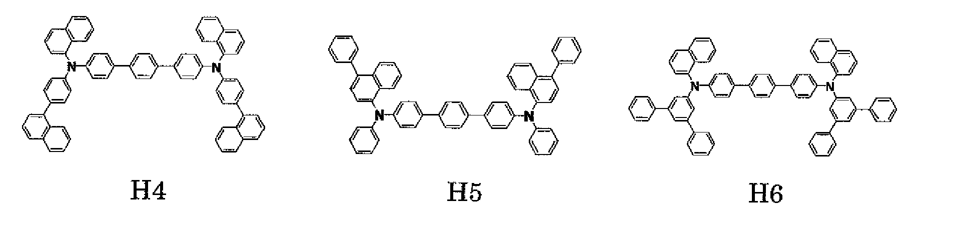

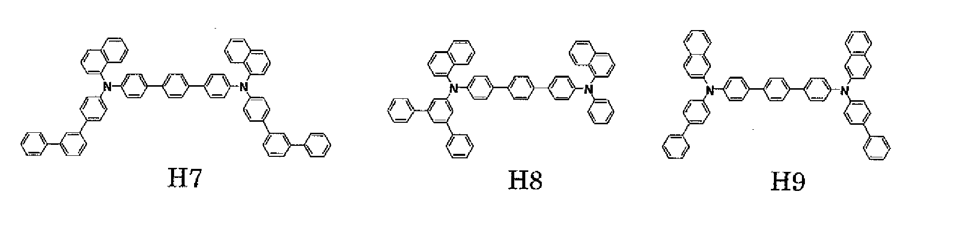

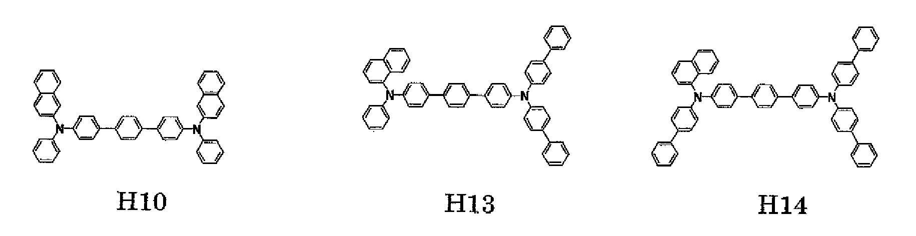

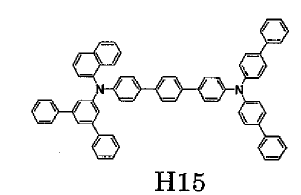

Following is a description about the organic EL device of the present invention.
The organic EL device of the present invention is preferably composed of one or more organic thin film layers including at least one light emitting layer and sandwiched between a cathode and an anode, wherein at least one of the organic thin film layers contains the aromatic amine derivative singly or in combination of two or more.
It is preferable that the organic thin film layer in the organic EL device of the present invention includes a hole transporting layer and the hole transporting layer contains the aromatic amine derivative of the present invention singly or in combination of two or more. Further, it is preferable that the hole transporting layer contains the aromatic amine derivative of the present invention as its main component.

Furthermore, it is preferable that the light emitting layer in the organic EL device of the present invention contains an arylamine compound and/or a styrylamine compound.
Examples of the arylamine compound include compounds represented by the following general formula (I) and examples of the styryl amine compound include compounds represented by the following general formula (II).

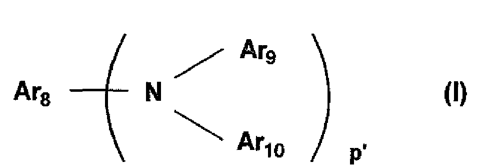

In the general formula (I), Ar₈ represents a group selected from a phenyl group, a biphenyl group, a terphenylyl group, a stilbene group and a distyrylaryl group; Ar₉ and Ar₁₀ each independently represents a hydrogen atom or an aromatic group having 6 to 20 carbon atoms; each of Ar₉ to Ar₁₀ may be substituted; and p' represents an integer of 1 to 4. It is further preferable that Ar₉ and/or Ar₁₀ is substituted by styryl group.
The aromatic group having 6 to 20 carbon atoms is preferably a phenyl group, a naphthyl group, an anthranyl group, a phenanthryl group, a terphenyl group or so.

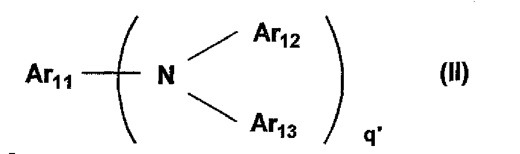

In the general formula (II), Ar₁₁ to Ar₁₃ each independently represents an aryl group having 5 to 40 ring carbon atoms that may be substituted; and q' represents an integer of 1 to 4.
The aryl group having 5 to 40 ring carbon atoms is preferably a phenyl group, a naphthyl group, an anthranyl group, a phenanthryl group, a pyrenyl group, a coronyl group, a biphenyl group, a terphenylyl group, a pyrrolyl group, a furanyl group, a thiophenyl group, a benzothiophenyl group, an oxadiazolyl group, a diphenyl anthranyl group, an indolyl group, a carbazolyl group, a pyridyl group, a benzoquinolyl group, a fluoranthenyl group, an acenaphthofluoranthenyl group, a stilbene group or so. The aryl group having 5 to 40 ring atoms may be substituted with a substituent, and preferable examples of the substituent include an alkyl group having 1 to 6 carbon atoms (an ethyl group, a methyl group, an i-propyl group, a n-propyl group, a s-butyl group, a t-butyl group, a pentyl group, a hexyl group, a cyclopentyl group, a cyclohexyl group, etc.); an alkoxy group having 1 to 6 carbon atoms (an ethoxy group, a methoxy group, an i-propoxy group, a n-propoxy group, a s-butoxy group, a t-butoxy group, a pentoxy group, a hexyloxy group, a cyclo pentoxy valve group, a cyclohexyl oxy group, etc.); an aryl group having 5 to 40 ring atoms; an amino group substituted with an aryl group having 5 to 40 ring atoms; an ester group which has an aryl group having 5 to 40 ring atoms; an ester group which has an alkyl group having 1 to 6 carbon atoms; a cyano group; a nitro group; and a halogen atom (a chlorine atom, a bromine atom, an iodine atom, etc.).

It is preferable that the aromatic amine derivatives of the present invention are employed especially as materials for the organic EL devices that emit blue light.

Following is a description regarding a device structure about the organic EL device of the present invention.

### (I) Construction of the organic EL device

Typical examples of the construction in the organic EL device of the present invention are shown below.
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) An anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode.
Among the above constructions, construction (8) is usually preferable though not limited to.
Although the aromatic amine derivative of the present invention may be employed for any of the above organic thin layers in the organic EL devices, it is contained preferably in a light emitting region or a hole transporting region, more preferably in the hole transporting region, and particularly preferably in the hole transporting layer, because the tendency for molecular crystallization is reduced and the yield in fabricating the organic EL devices is enhanced.
In the organic EL device of the present invention, the organic thin film layer preferably contains aromatic amine derivative of the present invention in an amount of 30 to 100 % by mole.

### (II) Substrate which transmits light

In general, the organic EL device is fabricated on a substrate which transmits light. The substrate which transmits light is a substrate for supporting the organic EL device and preferably a flat and smooth substrate having a light transmittance of 50 % or greater to visible light of 400 to 700 nm.
As the substrate which transmits light, for example, glass plate and synthetic resin plate are advantageously employed. Specific examples of the glass plate include soda ash glass, glass containing barium and strontium, lead glass, aluminosilicate glass, borosilicate glass, barium borosilicate glass and quartz. Specific examples of the synthetic resin plate include plate made of polycarbonate resins, acrylic resins, polyethylene telephthalate resins, polyether sulfide resins and polysulfone resins.

### (III) Anode

The anode in the organic EL device of the present invention has a function of injecting holes into a hole transporting layer or a light emitting layer, and it is effective that the anode has a work function of 4.5 eV or greater. Specific examples of the material for the anode include indium tin oxide alloy (ITO), tin oxide (NESA), indium-zinc oxide alloy (IZO), gold, silver, platinum, copper, etc.
The anode can be prepared by forming a thin film of the electrode material described above in accordance with a process such as the vapor deposition process and the sputtering process.
When the light emitted from the light emitting layer is obtained through the anode, it is preferable that the anode has a transmittance of the emitted light greater than 10 %. It is also preferable that the sheet resistivity of the anode is several hundreds Ω/□ or smaller. The thickness of the anode is, in general, selected usually in the range of from 10 nm to 1 µm and preferably in the range of from 10 to 200 nm.

### (IV) Light emitting layer

In the organic EL device of the present invention, the light emitting layer combines the following functions (1) to (3):
(1) The injecting function: the function of injecting holes from the anode or the hole injecting layer and injecting electrons from the cathode or the electron injecting layer when an electric field is applied;
(2) The transporting function: the function of transporting the injected charges (electrons and holes) by the force of the electric field; and
(3) The light emitting function: the function of providing the field for recombination of electrons and holes and promote the recombination to emit light.
Although there may be a difference between the capability of the holes being injected and the capability of the electrons being injected, and although there may be a difference between the transporting functions expressed by mobilities of the holes and the electrons, either one of the charges is preferable to be transferred.
As the process for forming the light emitting layer, a well-known process such as the vapor deposition process, the spin coating process and the LB process can be employed. It is particularly preferable for the light emitting layer to be a molecular deposit film. The molecular deposit film is a thin film formed by the deposition of a material compound in the gas phase or a thin film formed by the solidification of a material compound in a solution or liquid phase. In general, the molecular deposit film can be distinguished from the thin film formed in accordance with the LB process (the molecular accumulation film) based on the differences in the aggregation structure and higher order structures and functional differences caused by these structural differences.
In addition, as disclosed in JP 57-51781A, the light emitting layer can also be formed by dissolving a binder such as a resin and the material compounds into a solvent to prepare a solution, followed by forming a thin film from the prepared solution in accordance with the spin coating process or the like.
In the present invention, any well-known light emitting material other than the aromatic amine derivative of the present invention may be contained in the light emitting layer, or a light emitting layer containing any other well-known light emitting material may be laminated with the light emitting layer containing the aromatic amine derivative of the present invention, as long as the object of the present invention is not adversely affected.

Light emitting materials or doping materials to be used for the light emitting layer together with the aromatic amine derivatives of the present invention includes, for example, anthracene, naphthalene, phenanthrene, pyrene, tetracene, coronene, chrysene, fluorescein, perylene, phthaloperylene, naphthaloperylene, perinone, phthaloperinone, naphthaloperinone, diphenylbutadiene, tetraphenylbutadiene, coumarin, oxadiazole, aldazine, bisbenzoxazoline, bisstyryl, pyrazine, cyclopentadiene, quinoline metal complex, aminoquinoline metal complex, benzoquinoline metal complex, imine, diphenylethylene, vinylanthracene, diaminecarbazol, pyran, thiopyran, polymethyne, merocyanine, imidazol chelate oxinoid compound, quinacridone, rubrene and fluorescent dye, but not limited thereto.

Preferable host materials to be used for the light emitting layer together with the aromatic amine derivatives of the present invention include compounds represented by the following general formulae (i) to (ix).
An asymmetric anthracene represented by the following general formula (i): 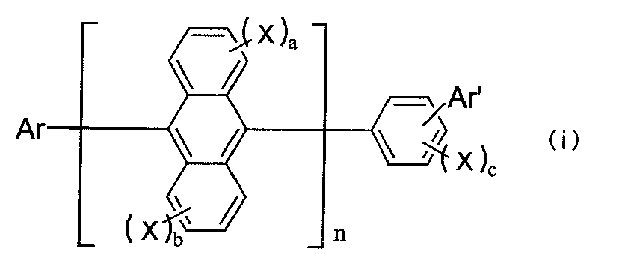 wherein Ar represents a substituted or unsubstituted fused aromatic group having 10 to 50 ring carbon atoms; Ar' represents a substituted or unsubstituted aromatic group having 6 to 50 ring carbon atoms; X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group or a hydroxyl group; a, b and c each independently represents an integer of 0 to 4; and n represents an integer of 1 to 3 with the proviso that when n is an integer of 2 or greater, the plural groups within square brackets ([ ]) is the same or different.

An asymmetric monoanthracene derivative represented by the following general formula (ii): 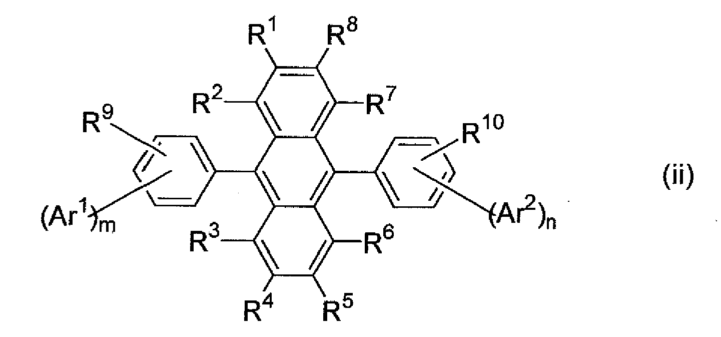 wherein Ar¹ and Ar² each independently represents a substituted or unsubstituted aromatic ring group having 6 to 50 ring carbon atoms; m and n each represents an integer of 1 to 4 with the proviso that in a case where m=n=1 and each bonding position of Ar¹ and Ar² to a benzene ring is bilaterally symmetric to each other, Ar¹ is different from Ar², and in a case where m or n represents an integer of 2 to 4, m is different from n; R¹ to R¹⁰ each independently represents a hydrogen atom, a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group.

An asymmetric pyrene derivative represented by the following general formula (iii): 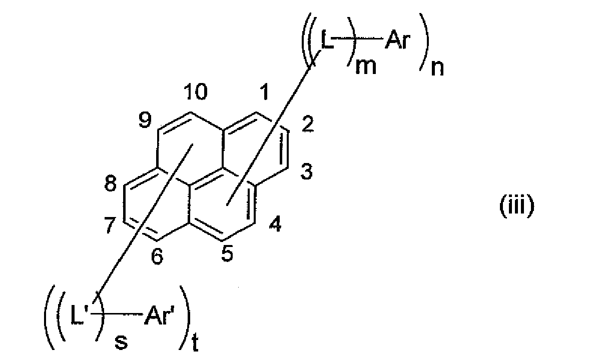 wherein Ar and Ar' each represents a substituted or unsubstituted aromatic group having 6 to 50 ring carbon atoms; L and L' each represents a substituted or unsubstituted phenylene group, a substituted or unsubstituted naphthalenylene group, a substituted or unsubstituted fluorenylene group or a substituted or unsubstituted dibenzosilolylene group; m represents an integer of 0 to 2, n represents an integer of 1 to 4, s represents an integer of 0 to 2 and t represents an integer of 0 to 4; L or Ar is bonded to any one of 1- to 5-positions of pyrene ring; and L' or Ar' is bonded to any one of 6- to 10-positions of pyrene ring; with the proviso that when n+t represents an even number, Ar, Ar', L and L' satisfy the following conditions (1) or (2):
(1) Ar ≠ Ar' and/or L ≠ L' (wherein ≠ means that each group has a different structure)
(2) when Ar = Ar' and L = L'
(2-1) m ≠ s and/or n ≠ t, or
(2-2) when m = s and n = t,
(2-2-1) L and L', or the pyrene ring, are respectively bonded to different positions of Ar and Ar', or
(2-2-2) in the case where L and L', or the pyrene ring, are respectively bonded to the same position of Ar and Ar', the case where L and L', or Ar and Ar' are bonded to 1- and 6-positions or 2- and 7-positions of the pyrene ring is excluded.

An asymmetric anthracene derivative represented by the following general formula (iv): 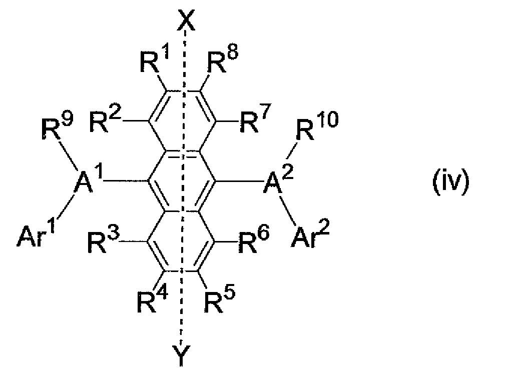 wherein A¹ and A² each independently represents a substituted or unsubstituted fused aromatic ring group having 10 to 20 ring carbon atoms; Ar¹ and Ar² each independently represents a hydrogen atom, or a substituted or unsubstituted aromatic ring group having 6 to 50 ring carbon atoms; R¹ to R¹⁰ each independently represents a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group; and the number of each of Ar¹, Ar², R⁹ and R¹⁰ may be two or more, and two neighboring groups thereof may form a saturated or unsaturated ring structure; with the proviso that the groups at 9- and 10-positions of the central anthracene are not symmetrical with respect to the X-Y axis.

An anthracene derivative represented by the following general formula (v): 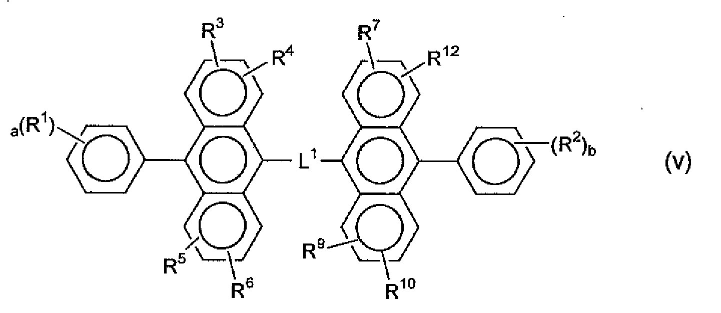 wherein R¹ to R¹⁰ each independently represents a hydrogen atom, an alkyl group, a cycloalkyl group, an aryl group which may be substituted, an alkoxyl group, an aryloxy group, an alkylamino group, an alkenyl group, an arylamino group or a heterocyclic group which may be substituted; a and b each represents an integer of 1 to 5, and when each of a and b is 2 or greater, R¹'s or R²'s may be the same or different, and R¹'s or R²'s may bond each other to form a ring; each pair of R³ and R⁴, R⁵ and R⁶, R⁷ and R⁸, and R⁹ and R¹⁰ may bond each other to form a ring, L¹ represents a single bond, -O-, -S-, -N(R)-, an alkylene group or an arylene group wherein R represents an alkyl group or an aryl group which may be substituted.

An anthracene derivative represented by the following general formula (vi): 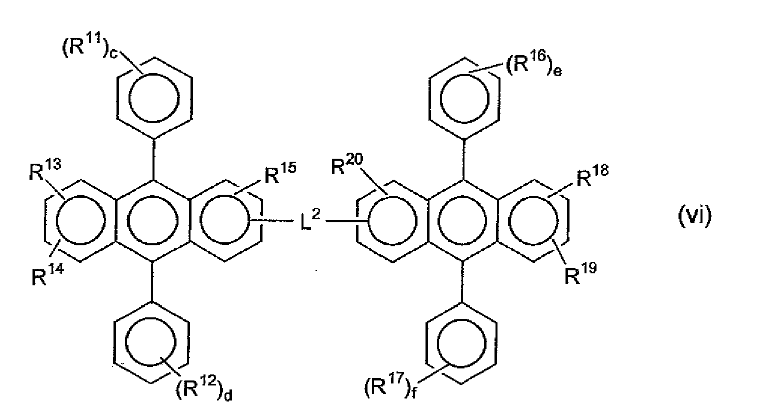 wherein R¹¹ to R²⁰ each independently represents a hydrogen atom, an alkyl group, a cycloalkyl group, an aryl group, an alkoxyl group, an aryloxy group, an alkylamino group, an arylamino group or a heterocyclic group which may be substituted; c, d, e and f each represents an integer of 1 to 5, and when each of c, d, e and f is 2 or greater, R¹¹'s, R¹²'s, R¹⁶'s or R¹⁷'s may be the same or different, and R¹¹'s, R¹²'s, R¹⁶'s or R¹⁷'s may bond each other to form a ring; each pair of R¹³ and R¹⁴, and R¹⁸ and R¹⁹ may bond each other to form a ring; L² represents a single bond, -O-, -S-, -N(R)-, an alkylene group or an arylene group wherein R represents an alkyl group or an aryl group which may be substituted.

A spirofluorene derivative represented by the following general formula (vii): 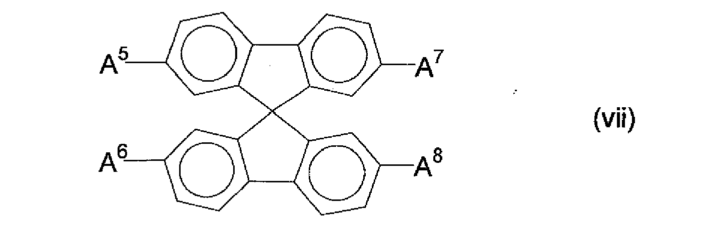 wherein A⁵ to A⁸ each independently represents a substituted or unsubstituted biphenylyl group or a substituted or unsubstituted naphthyl group.

A compound containing a fused ring represented by a following general formula (viii): 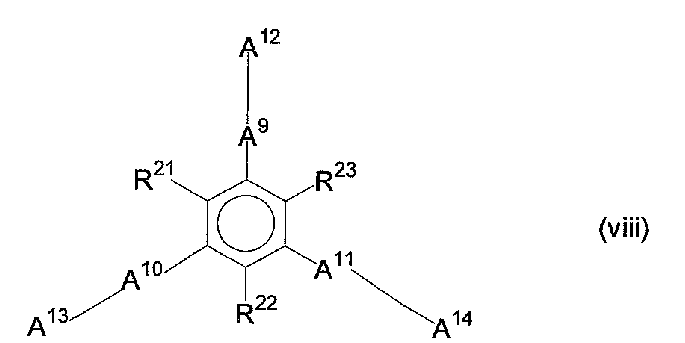 wherein A⁹ to A¹⁴ are same as described above; R²¹ to R²³ each independently represents a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms or a halogen atom; and at least one of A⁹ to A¹⁴ represents a fused aromatic ring having 3 or more rings.

A fluorene compound represented by the following general formula (ix): 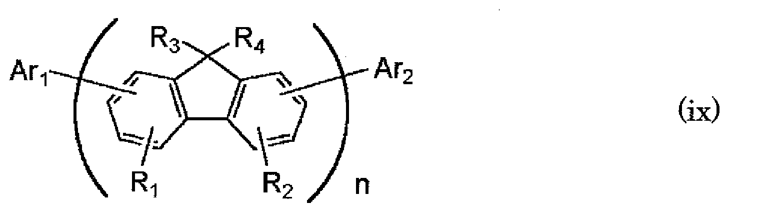 wherein R₁ and R₂ each independently represents a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group or a halogen atom; R₁'s and R₂'s bonding to different fluorene groups may be respectively the same or different, and R₁ and R₂ bonding to the same fluorene group may be the same or different; R₃ and R₄ each independently represents a hydrogen atom, a substituted or unsubstituted alkyl group, a substituted or unsubstituted aralkyl group, a substituted or unsubstituted aryl group, or a substituted or unsubstituted heterocyclic group, and R₃'s and R₄'s bonding to different fluorene groups may be respectively the same or different, and R₃ and R₄ bonding to the same fluorene group may be the same or different; Ar₁ and Ar₂ may be the same or different and each independently represents a substituted or unsubstituted fused polycyclic aromatic group having 3 or more benzene rings or a substituted or unsubstituted fused polycyclic heterocyclic group which has 3 or more benzene rings and hetero rings in total and is bonded to the fluorene group via carbon atom; and n represents an integer of 1 to 10.

Among the above host materials, the anthracene derivative is preferable, the monoanthracene derivative is more preferable, and the asymmetric anthracene is particularly preferable.
In addition, a phosphorescent compound may be employed as a light emitting material for dopant. As the host material, a compound containing a carbazole ring is preferably used. The dopant is not limited as long as it is a a compound capable of emitting light from triplet exciton, and preferably a metal complex containing at least one metal selected from the group consisting of Ir, Ru, Pd, Pt, Os and Re, more preferably a porphyrin metal complex or an ortho-metallated complex.
A suitable host for phosphorescence composed of a compound containing a carbazole ring is a compound having a function of making the phosphorescent compound to emit light by the energy transfer from its excitation state to the phosphorescent compound. The host compound is not limited as long as capable of transferring the exciton energy to the phosphorescent compound and may be appropriately selected according to the purpose. The host compound may have any group such as a hetero ring in addition to the carbazole ring.

Specific examples of the host compound include a carbazole derivative, a triazole derivative, an oxazole derivative, an oxadiazole derivative, an imidazole derivative, a polyarylalkane detivative, a pyrazoline derivative, a pyrazlone derivative, a phenylenediamine derivative, an arylamine derivative, a calcone derivative substituted by amino group, a styrylanthracene derivative, a fluorenone derivative, a hydrazone derivative, a stilbene derivative, a silazane derivative, an aromatic tertiary amine compound, a styrylamine compound, an aromatic dimethylidene compound, a porphyrin-based compound, an anthraquinodimethane derivative, an anthrone derivative, a diphenylquinone derivative, a thiopyrandioxide derivative, a carbodimide derivative, a fluorenylidene methane derivative, a distyrylpyrazine derivative, heterocyclic tetracarboxylic anhydride such as a naphthaleneperylene, a phthalocyanine derivative, various metal complex polysilane compound such as a metal complex of 8-quinolinol derivative and a metal complex having a ligand of metallophthalocyanine, benzoxazole or benzothiazole, an electrically conductive polymeric oligomer such as a poly(N-vinylcarbazole) derivative, an aniline copolymer, a thiophene oligomer and a polythiophene, high-molecular compound such as a polythiophene derivative, a polyphenylene derivative, a polyphenylenevinylene derivative and a polyfluorene derivative. The host compound may be used alone or in combination of two or more.
More specific examples include the following:

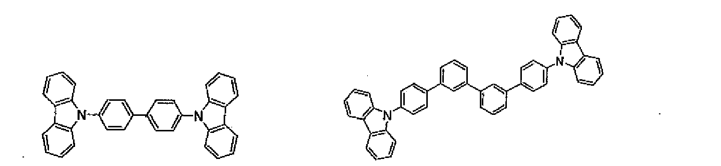

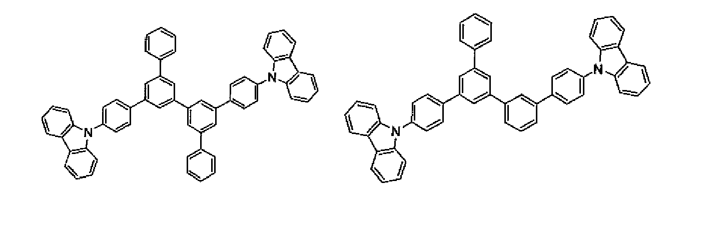

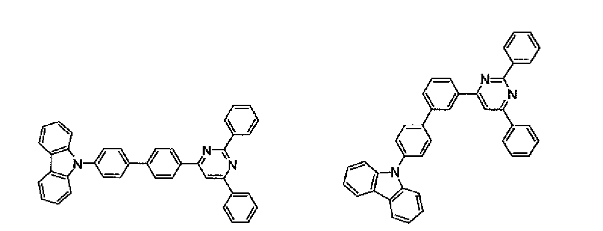

The phosphorescent dopant is a compound capable of emitting light from the triplet exciton. The phosphorescent dopant is not restricted as long as it emits light from the triplet exciton, and preferably a metal complex containing at least one metal selected from the group consisting of Ir, Ru, Pd, Pt, Os and Re, more preferably a porphyrin metal complex or an ortho-metallated metal complex. As the porphyrin metal complex, a porphyrin platinum complex is preferable. The phosphorescent compound may be used alone or in combination of two more.
There are various ligands to form the ortho-metallated metal complex, and preferred are 2-phenylpyridine derivatives, 7,8-benzoquinoline derivatives, 2-(2-thienyl)pyridine derivatives, 2-(1-naphthyl)pyridine derivatives, and 2-phenylquinoline derivatives. The derivatives may have a substituent as occasion demands. In particular, a dopant introduced with a fluorine atom or a trifluoromethyl group is preferable for the blue light emission. In addition, a ligand other than the above ligands such as acetylacetonate and picric acid may be introduced as a co-ligand.
The amount of the phosphorescent dopant in the light emitting layer may be appropriately selected without particular limitation, and for example, it may be from 0.1 to 70 % by mass, preferably from 1 to 30 % by mass. The emission is faint and the effect of use is not obtained when the amount is less than 0.1 % by mass. The concentration quenching becomes noticeable so that the device performance is deteriorated when the amount exceeds 70 % by mass.
Further, the light emitting layer may contain a hole transporting material, a electron transporting material or a polymer binder, if necessary.
The thickness of the light emitting layer is, in general, selected in the range of from 5 to 50 nm, preferably in the range of from 7 to 50 nm and the most preferably in the range of from 10 to 50 nm. It is resulted in difficult to form the light emitting layer and to control chromaticity thereof when the thickness is thinner than 5 nm, and it may be resulted in possibility of elevating driving voltage when it exceeds 50 nm.

### (V) Hole injecting and transporting layer (hole transporting region)

The hole injecting and transporting layer is a layer which helps the injection of holes into the light emitting layer and transports the holes to the light emitting region. The layer exhibits a great mobility of holes and, in general, has an ionization energy as small as 5.5 eV or smaller. For the hole injecting and transporting layer, a material which transports holes to the light emitting layer at a small strength of the electric field is preferable. A material which exhibits, for example, a mobility of holes of at least 10⁻⁴ cm²/V·sec under an electric field of from 10⁴ to 10⁶ V/cm is preferable.
When the aromatic amine derivative of the present invention is employed in the hole transporting region, the hole injecting and transporting layer may be composed of the aromatic amine derivative of the present invention alone or in combination with another material.
With regard to the material which may be employed for forming the hole injecting and transporting layer in combination with the aromatic amine derivative of the present invention, any material having the foregoing preferable properties is employed without particularly restricted, which is selected from compounds commonly used as a hole transporting material of photoconductive materials and compounds used for forming the hole injecting and transporting layer of EL devices.

Specific examples include triazole derivatives (refer to U.S. Pat. No. 3,112,197, etc.), oxadiazole derivatives (refer to U.S. Pat. No. 3,189,447, etc.), imidazole derivatives (refer to JP-B 37-16096, etc.), polyarylalkane derivatives (refer to U.S. Pat. Nos. 3,615,402; 3,820,989; 3,542,544, JP-B 45-555, JP-B 51-10983, JP 51-93224A, JP 55-17105A, JP 56-4148A, JP 55-108667A, JP 55-156953A, JP 56-36656A, etc.), pyrazoline derivatives and pyrazolone derivatives (refer to U.S. Pat. Nos. 3,180,729; 4,278,746; JP 55-88064A, JP 55-88065A, JP 49-105537A, JP 55-51086A, JP 56-80051A, JP 56-88141A, JP 57-45545A, JP 54-112637A, JP 55-74546A, etc.), phenylenediamine derivatives (refer to U.S. Pat. No. 3,615,404; JP-B 51-10105, JP-B 46-3712, JP-B 47-25336, JP 54-53435A, JP 54-110536A, JP 54-119925A, etc.), arylamine derivatives (refer to U.S. Pat. Nos. 3,567,450; 3,180,703; 3,240,597; 3,658,520; 4,232,103; 4,175,961; 4,012,376; JP-B 49-35702, JP-B 39-27577, JP 55-144250A, JP 56-119132A, JP 56-22437A, German Patent No. 1,110,518, etc.), amino-substituted chalcone derivatives (refer to U.S. Pat. No. 3,526,501, etc.), oxazole derivatives (disclosed in U.S. Pat. No. 3,257,203, etc.), styrylanthracene derivatives (refer to JP 56-46234A, etc.), fluorenone derivatives (refer to JP 54-110837A, etc.), hydrazone derivatives (Refer to U.S. Pat. No. 3,717,462, JP 54-59143A, JP 55-52063A, JP 55-52064A, JP 55-46760A, JP 55-85495A, JP 57-11350A, JP 57-148749A, JP 2-311591A, etc.), stilbene derivatives (refer to JP 61-210363A, JP 61-228451A, JP 61-14642A, JP 61-72255A, JP 62-47646A, JP 62-36674A, JP 62-10652A, JP 62-30255A, JP 60-93455A, JP 60-94462A, JP 60-174749A, JP 60-175052A, etc.), silazane derivatives (U.S. Pat. No. 4,950,950), polysilane-based polymer (JP 2-204996A), aniline-based copolymer (JP 2-282263A), an electrically conductive high-molecular oligomer disclosed in JP 1-211399 A (particularly, thiophene oligomer), etc.

With regard to the material for the hole injecting and transporting layer, the above materials are also employable, and porphyrin compounds (disclosed in JP 63-295695A), aromatic tertiary amine compounds and styryl amine compounds (refer to U.S Pat. No. 4,127,412, JP 53-27033A, JP 54-58445A, JP 54-149634A, JP 54-64299A, JP 55-79450A, JP 55-144250A, JP 56-119132A, JP 61-295558A, JP 61-98353A, JP 63-295695A, etc.) are preferable and the aromatic tertiary amine compounds are particularly preferable.
Further examples include, 4,4'-bis(N-(1-naphthyl) -N-phenylamino)biphenyl (NPD) which has 2 fused aromatic rings in its molecule described in U.S Pat. No. 5,061,569 and 4,4',4"-tris (N-(3-methylphenyl)-N-phenylamino)triphenylamine (MTDATA) described in JP 4-308688A which includes three triphenylamine units connected in a star burst configuration.
In addition to the above-mentioned aromatic dimethylidene compound described as a material for the light emitting layer, inorganic compound such as p-type Si and p-type SiC may be used as the material for the hole injecting and transporting layer.

To form the hole injecting and transporting layer, a thin film may be formed from the aromatic amine derivative of the present invention in accordance with a well-known process such as the vacuum vapor deposition process, the spin coating process, the casting process and the LB process. Although the thickness of the hole injecting and transporting layer is not particularly limited, the thickness is usually from 5 nm to 5 µm. The hole injecting and transporting layer may be a single layer made of one or more kinds of materials mentioned above or may be laminated with another hole injecting and transporting layer made of a different material, as long as the hole injecting and transporting layer contains the aromatic amine derivative of the present invention in its hole transporting region.
An organic semiconductor layer which preferably has an electric conductance of 10⁻¹⁰ S/cm or greater may be provided to assist the injection of holes or electrons into the light emitting layer. Examples of the materials for the organic semiconductor layer include electrically conductive oligomers such as an oligomer having thiophene and an oligomer having arylamine disclosed in JP 8-193191A; and electrically conductive dendrimers such as a dendrimer having an arylamine dendrime.

### (VI) Electron injecting and transporting layer

The electron injecting and transporting layer is a layer having a great electron mobility, which assists the injection of electrons into the light emitting layer and transports them to a light emitting region. Among the electron injecting layers, the adhesion improving layer is a layer made of a material exhibiting excellent adhesion to the cathode.
Further, it is known that because the emitted light reflects on the electrode (cathode in this case) in the organic EL device, the light taken out directly through the anode and the light taken out after the reflection on the electrode interferes each other. To utilize the interference effect effectively, the thickness of the electron transporting layer is appropriately selected from several nm to several µm. When the film is thicker, the hole mobility is preferably at least 10⁻⁵ cm²/V·s under an electric field of from 10⁴ to 10⁶ V/cm for avoiding the elevation of driving voltage.
As the material for the electron injecting layer, metal complexes of 8-hydroxyquinoline or derivatives thereof and oxadiazole derivatives are preferable. Examples of the metal complexes of 8-hydroxyquinoline and derivatives thereof include metal chelate oxinoid compounds including chelates of oxine (in general, 8-quinolinol or 8-hydroxyquinoline), for example, tris(8-quinolinol)aluminum.

Examples of the oxadiazole derivatives include an electron transfer compound represented by the following general formulae: 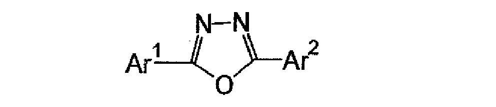 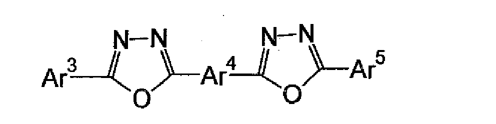 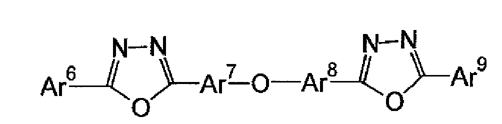 wherein Ar¹, Ar², Ar³, Ar⁵, Ar⁶ and Ar⁹ may be the same or different and each independently represents a substituted or unsubstituted aryl group; Ar⁴, Ar⁷ and Ar⁸ may be the same or different and each independently represents a substituted or unsubstituted arylene group.
Examples of the aryl group include a phenyl group, a biphenyl group, an anthryl group, a perilenyl group and a pyrenyl group. Examples of the arylene group include a phenylene group, a naphthylene group, a biphenylene group, an anthrylene group, a perilenylene group, a pyrenylene group, etc. Examples of the substituent include an alkyl group having 1 to 10 carbon atoms, an alkoxy group having 1 to 10 carbon atoms group and a cyano group. The electron transfer compound is preferably a thin-film forming compound.

Specific examples of the electron transfer compounds are shown below: 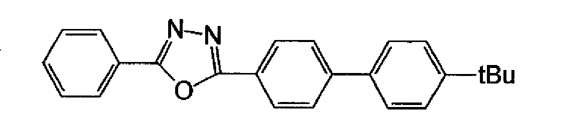 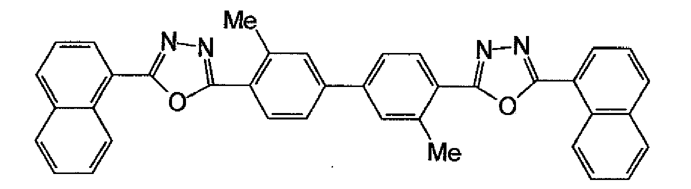 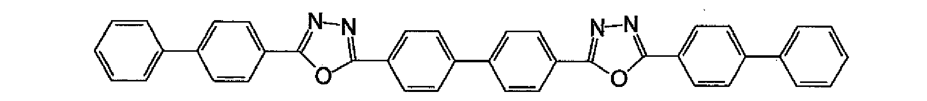 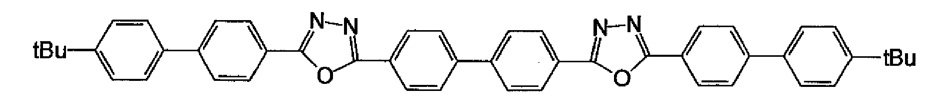 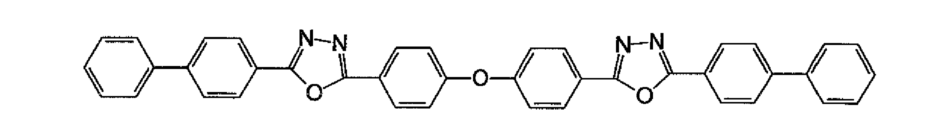 The compounds represented by the following general formulae (A) to (F) may be also used as the material for the electron injecting layer and the electron transporting layer.
A nitrogen-containing heterocyclic derivative represented by the following general formula (A) or (B):

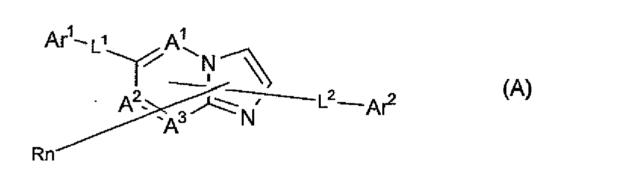

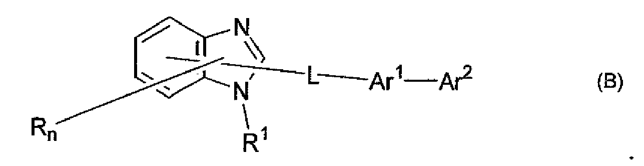

In the general formulae (A) and (B), A¹ to A³ each independently represents a nitrogen atom or a carbon atom.
Ar¹ represents a substituted or unsubstituted aryl group having 6 to 60 ring carbon atoms or a substituted or unsubstituted heteroaryl group having 3 to 60 ring carbon atoms; Ar² represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxy group having 1 to 20 carbon atoms or a divalent group derived from therefrom. At least one of Ar¹ and Ar² represents a substituted or unsubstituted fused ring group having 10 to 60 ring carbon atoms, a substituted or unsubstituted monohetero fused ring group having 3 to 60 ring carbon atoms or a divalent group derived therefrom.
L¹, L² and L each independently represents a single bond, a substituted or unsubstituted arylene group having 6 to 60 ring carbon atoms, a substituted or unsubstituted heteroarylene group having 3 to 60 ring carbon atoms or a substituted or unsubstituted fluorenylene group.
R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms; n represents an integer of 0 to 5; when n is 2 or greater, Rs may be the same or different and adjacent couple of Rs may bond to form a carbocyclic aliphatic ring or a carbocyclic aromatic ring.
R¹ represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxy group having 1 to 20 carbon atoms or -L-Ar¹-Ar².

A nitrogen-containing heterocyclic derivative represented by the following general formula (C):

HAr-L-Ar¹-Ar² (C)

wherein HAr represents a nitrogen-containing heterocyclic group having 3 to 40 carbon atoms which may have a substituent; L represents a single bond, an arylene group having 6 to 60 carbon atoms which may have a substituent, a heteroarylene group having 3 to 60 carbon atoms which may have a substituent or a fluorenylene group which may have a substituent; Ar¹ represents a divalent aromatic hydrocarbon group having 6 to 60 carbon atoms which may have a substituent; and Ar² represents an aryl group having 6 to 60 carbon atoms which may have a substituent or a heteroaryl group having 3 to 60 carbon atoms which may have a substituent.

A silacyclopentadiene derivative represented by the following general formula (D):

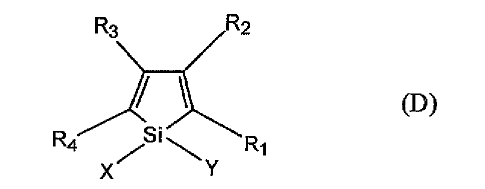

wherein X and Y each independently represents a saturated or unsaturated hydorocarbon group having 1 to 6 carbon atoms, an alkoxy group, an alkenyloxy group, an alkynyloxy group, a hydroxy group, a substituted or unsubstituted aryl group, or a substituted or unsubstituted hetero ring, or X and Y represents a saturated or unsaturated ring by bonding to each other; R₁ to R₄ each independently represents a hydrogen atom, a halogen atom, a substituted or unsubstituted alkyl group having 1 to 6 carbon atoms, an alkoxy group, an aryloxy group, a perfluoroalkyl group, a perfluoro alkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a hetero ring group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or an adjacent pair of R₁ to R₄ represents a substituted or unsubstituted fused ring.

A borane derivative represented by the following general formula (E):

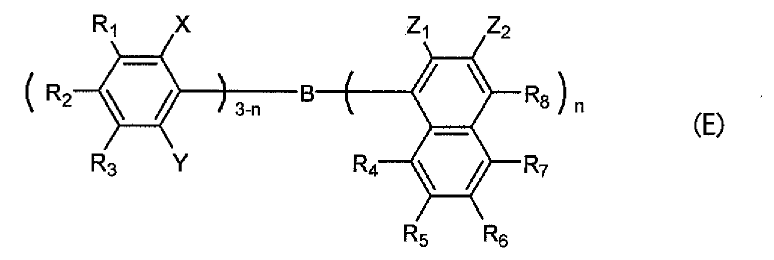

wherein R₁ to R₈ and Z₂ each independently represents a hydrogen atom, a saturated or unsaturated hydrocarbon group, an aromatic group, a hetero ring group, substituted amino group, a substituted boryl group, an alkoxy group or an aryloxy group; X, Y and Z₁ each independently represents a saturated or unsaturated hydorocarbon group, an aromatic group, a hetero ring group, substituted amino group, an alkoxy group or an aryloxy group; substituents of Z₁ and Z₂ may bonds to each other to form a fused ring; n represents an integer of 1 to 3; and when n is 2 or greater, Z₁'s may be different from each other, with the proviso that a case where n is 1, X, Y and R₂ are methyl groups and R₈ is a hydrogen atom or a substituted boryl group and a case where n is 3 and Z₁ is a methyl group are excluded.

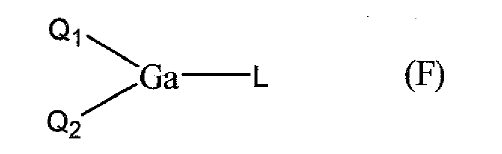

wherein Q¹ and Q² each independently represents a ligand represented by the following general formula (G), L represents a halogen atom, a substituted or unsubstituted alkyl group, a substituted or unsubstituted cycloalkyl group, a substituted or unsubstituted aryl group, a substituted or unsubstituted heterocyclic group, -OR¹ wherein R¹ represents a hydrogen atom, a substituted or unsubstituted alkyl group, a substituted or unsubstituted cycloalkyl group, a substituted or unsubstituted aryl group, a substituted or unsubstituted heterocyclic group, or a ligand represented by -O-Ga-Q³(Q⁴) wherein Q³ and Q⁴ are the same as Q¹ and Q².

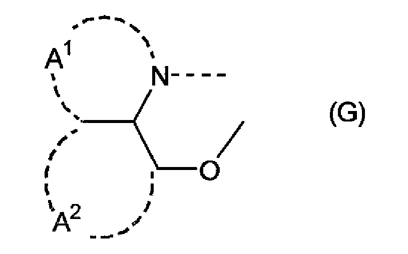

wherein A¹ and A² each represents a fused six-membered aryl ring structure which may be substituted.

The metal complex strongly characterizes n-type semiconductor and has a large capability of the electron injection. Since the generation energy for forming the metal complex is small, the bonding between the metal and the ligand is strong, to increase the fluorescence quantum efficiency of light emitting materias
Specific examples of the subsituents of rings A¹ and A² each forming the ligand in the general formula (G) include halogen atom such as chlorine atom, bromine atom, iodine atom and fluorine atom; substituted or unsubstituted alkyl group such as methyl group, ethyl group, propyl group, butyl group, s-butyl group, t-butyl group, pentyl group, hexyl group, heptyl group, octyl group, stearyl group, trichloromethyl group, etc.; substituted or unsubstituted aryl group such as phenyl group, naphthyl group, 3-methylphenyl group, 3-methoxyphenyl group, 3-fluorophenyl group, 3-trichloxomethylphenyl group, 3-trifluoromethylphenyl group, 3-nitrophenyl group, etc. substituted or unsubstituted alkoxy group such as methoxy group, n-butoxy group, t-butoxy group, trichloromethoxy group, trifluoroethoxy group, pentafluoropropoxy group, 2,2,3,3-tetrafluoropropoxy group, 1,1,1,3,3,3-hexafluoro-2-propoxy group, 6-(perfluoroethyl)hexyloxy group, etc.; substituted or unsubstituted aryloxy group such as phenoxy group, p-nitrophenoxy group, p-t-butylphenoxy group, 3-fluorophenoxy group, pentafluorophenyl group, 3-trifluoromethylphenoxy group, etc.; substituted or unsubstituted alkylthio group such as methylthio group, ethylthio group, t-butylthio group, hexylthio group, octylthio group, trifluoromethylthio group, etc.; substituted or unsubstituted arylthio group such as phenylthio group, p-nitrophenylthio group, p-t-butylphenylthio group, 3-fluorophenylthio group, pentafluorophenylthio group, 3-trifluoromethylphenylthio group, etc.; cyano group; nitro group; amino group; mono or di-substituted amino groups such as methylamino group, diethylamino group, ethylamino group, diethylamino group, dipropylamino group, dibutyl amino group, diphenylamino group, etc.; acylamino groups such as bis(acetoxymethyl)amino group, bis(acetoxyethyl)amino group, bis(acetoxypropyl)amino group, bis(acetoxybutyl) amino group, etc.; hydroxy group; siloxy group; acyl group; carbamoyl group such as methylcarbamoyl group, dimethylcarbamoyl group, ethylcarbamoyl group, diethylcarbamoyl group, a propylcarbamoyl group, butyl carbamoyl group, a phenylcarbamoyl group, etc.; carboxylic acid group; sulfonic acid group; imido group; cycloalkyl group such as cyclopentyl group, cyclohexyl group, etc.; aryl group such as phenyl group, naphthyl group, biphenyl group, anthryl group, phenanthryl group, fluorenyl group, pyrenyl group, etc.; heterocyclic group such as pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triazinyl group, carbazolyl group, furanyl group, thiophenyl group, oxazolyl group, an oxadiazolyl group, a benzoxazolyl group, a thiazolyl group, a thiadiazolyl group, benzothiazolyl group, triazolyl group, imidazolyl group, benzimidazolyl group, pranyl group, etc. The above substituents may bond each other to form a six-membered aryl ring or hetero ring.

A preferred embodiment of the organic EL device of the present invention contains a reductive dopant in an electron transporting region or an interfacial region between a cathode and an organic compound layer. The reductive dopant is defined as the substance capable of reducing an electron transporting compound. Accordingly, various compounds having a reducing property may be used, and examples of the reductive dopant include at least one compound selected from alkali metals, alkaline earth metals, rare earth metals, oxides of alkali metals, halides of alkali metals, oxides of alkaline earth metals, halides of alkaline earth metals, oxides of rare earth metals, halides of rare earth metals, organic complexes of alkali metals, organic complexes of alkaline earth metals, and organic complexes of rare earth metals.
Examples of the preferable reductive dopant include at least one alkali metal selected from the group consisting of Na (work function: 2.36 eV), K (work function: 2.28 eV), Rb (work function: 2.16 eV) and Cs (work function: 1.95 eV) or at least one alkaline earth metals selected from the group consisting of Ca (work function: 2.9eV), Sr (work function: 2.0 to 2.5 eV) and Ba (work function: 2.52eV). A reductive dopant having a work function of 2.9 eV or smaller is particularly preferable. Among those, more preferable reductive dopants include at least one alkali metal selected from the group consisting of K, Rb and Cs, more preferably Rb or Cs and most preferably Cs. Since those alkali metals have a particularly high reducing capability, the luminance is improved and the lifetime is prolonged by the addition thereof into an electron injection region in a relatively small amount. A combination of two or more alkali metals is also preferably used as the reductive dopant having a work function of 2.9 eV or smaller. A combination containing Cs such as Cs and Na, Cs and K, Cs and Rb and Cs, Na and K is particularly preferred.
By combinedly containing Cs, the reducing capability is effectively performed, and the luminance is enhaced and the lifetime is prolonged by the addition into the electron injection region.

In the organic EL device of the present invention, an electron injecting layer made of an electrically insulating material or a semiconductor may be further disposed between the cathode and the organic layer. The electron injecting layer enables to effectively prevent a leak of electric current and to improve the electron injection property. The electric insulator is preferably at least one metal compound selected from the group consisting of alkali metal chalcogenide, alkaline earth metal chalcogenide, halide of alkali metal and halide of alkaline earth metal. When the electron injecting layer is made of these alkali metal chalcogenide or so, the electron injection property is further improved. Preferable examples of the alkali metal chalcogenide include Li₂O, K₂O, Na₂S, Na₂Se and Na₂O. Preferable examples of the alkaline earth metal chalcogenide include CaO, BaO, SrO, BeO, BaS and CaSe. Preferable examples of the alkali metal halide include LiF, NaF, KF, LiCl, KCl and NaCl. Preferable examples of the alkaline earth metal halide include fluorides such as CaF₂, BaF₂, SrF2, MgF₂ and BeF₂ and halides other than the fluorides.
Examples of the semiconductor for constituting the electron transporting layer include oxides, nitrides and oxynitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound for constituting the electron transporting layer is in the form of a crystallite or amorphous insulating thin film. When the electron transporting layer is constituted of the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

### (VII) Cathode

The cathode is formed from an electrode substance such as metal, alloy, electrically conductive compound or a mixture thereof each having a small work function (4 eV or smaller) to ensure the electron injection into the electron injecting or transporting layer or a light emitting layer. Examples of the electrode substance include sodium, sodium-potassium alloy, magnesium, lithium, magnesium-silver alloy, aluminum/aluminum oxide, aluminum-lithium alloy, indium, rare earth metal, etc.
The cathode is prepared by forming a thin film of the electrode material described above in accordance with a process such as the vapor deposition process and the sputtering process.
When the light emitted from the light emitting layer is taken out of the cathode, it is preferable that the cathode has a transmittance of greater than 10 % to the emitted light.
It is also preferable that the sheet resistivity of the cathode is several hundreds Ω/□ or smaller and the thickness of the cathode is, in general, from 10 nm to 1 µm and preferably from 50 to 200 nm.

### (VIII) Insulating layer

In general, an organic EL device tends to form defects in pixels due to leak and short circuit, because an electric field is applied to ultra-thin films. To prevent the defects, an insulating thin film layer is preferably inserted between the pair of electrodes.
Examples of the material for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be employed.

### (IX) Fabrication process of the organic EL device

The organic EL device of the present invention is fabricated, for example, by forming an anode, a light emitting layer, an optional hole injecting and transporting layer, an optional electron injecting and transporting layer, and a cathode in accordance with the process using the materials each being described above. Alternatively, each layer may be formed in a reverse order from the cathode to the anode.
An embodiment of the fabrication of an organic EL device having a construction of anode/hole injecting layer/light emitting layer/electron injecting layer/cathode in this order on a light-transmitting substrate will be described in the following.
First, on a suitable light-emitting substrate, a thin film of an anode substance is formed so as to have a film thickness of 1 µm or thinner, preferably from 10 nm to 200 nm in accordance with a vapor deposition process, a sputtering process, etc. Then, a hole injecting layer is formed on the anode. The hole injecting layer can be formed in accordance with the vacuum vapor deposition process, the spin coating process, the casting process or the LB process, as described above. The vacuum vapor deposition process is preferable because a uniform film can be easily obtained and pinhole is little formed. When the hole injecting layer is formed in accordance with the vacuum vapor deposition process, the conditions are preferably selected from the following ranges: temperature of deposition source: 50 to 450°C; degree of vacuum: 10⁻⁷ to 10⁻³ Torr; vapor deposition rate: 0.01 to 50 nm/s; temperature of substrate: -50 to 300°C; and film thickness: 5 nm to 5 µm; although depending on the employed compound (material for hole injecting layer), the crystal structure and the recombination structure.

Subsequently, the light emitting layer is formed on the hole injecting layer by depositing a thin film of the organic light emitting material in accordance with the vacuum vapor deposition process, the sputtering process, the spin coating process or the casting process. The vacuum vapor deposition process is preferable because a uniform film can be easily obtained and pinhole is little formed. When the light emitting layer is formed in accordance with the vacuum vapor deposition process, the conditions of the vacuum vapor deposition can be selected in the same ranges as in the deposition of the hole injecting layer, although depending on the compound to be used.
Next, the electron injecting layer is formed on the light emitting layer. Similarly to the formation of the hole injecting layer and light emitting layer, the electron injecting layer is preferably formed in accordance with the vacuum vapor deposition process, because a uniform film is required. The conditions of the vacuum vapor deposition can be selected from the same ranges as in the formation of the hole injecting layer and light emitting layer.
The aromatic amine derivative of the present invention may be vapor-deposited together with another material, although depending on the region, light emitting region or hole transporting region, to which the aromatic amine derivative is contained. When the spin coating process is employed, the aromatic amine derivative is made into the layer by blending it with another material.
Finally, the cathode is formed on the electron injecting layer, to obtain an organic EL device.
The cathode is made of a metal and can be formed in accordance with the vacuum vapor deposition process or the sputtering process. However, the vacuum vapor deposition process is preferably employed in order to prevent the underlying organic layers from being damaged during the formation of the film.
In the above fabrication of the organic EL device, the layers from the anode to the cathode are successively formed preferably in a single evacuation operation.

The process for forming the layers in the organic EL device of the present invention is not particularly limited. A known process such as the vacuum vapor deposition process and the spin coating process or so can be employed. The organic thin film layer containing the compound of the formula (1) included in the organic EL device of the present invention can be formed in accordance with the vacuum vapor deposition process, the molecular beam epitaxy process (the MBE process) or a known method of coating a solution of the compound such as the dipping process, the spin coating process, the casting process, the bar coating process and the roller coating process.
The thickness of each layer in the organic thin film layer of the organic EL device of the present invention is not particularly limited. In general, an excessively thin layer tends to have defects such as pinholes, and an excessively thick layer requires a high applied voltage to reduce the efficiency. Therefore, the thickness is preferably from several nm to 1 µm.
The organic EL device emits light when a direct voltage of 5 to 40 V is applied with the anode being + terminal and the cathode being - terminal. In the reverse polarity, no electric current flows and no light is emitted upon the application of voltage. When an alternating voltage is applied, the uniform light emission is observed only in the polarity where the anode is + and the cathode is -. The wave shape of alternating voltage in not limited.

### EXAMPLES

Next, the present invention is described in more detail by reference to the following synthesis examples and examples.
The structural formulae of intermediate compounds prepared in Synthesis Examples 1 to 3 are as follows.

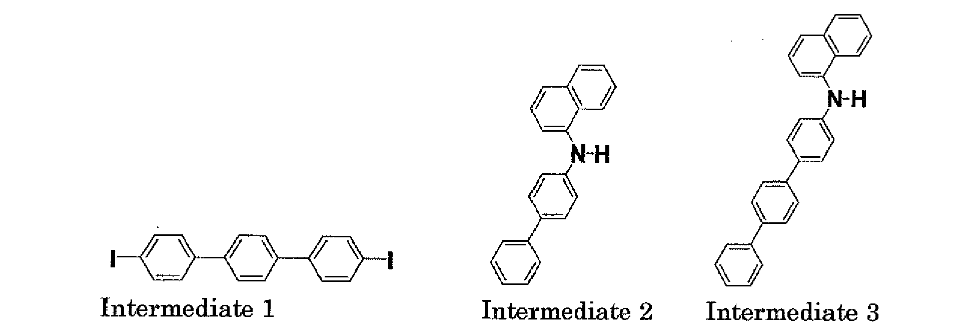

### Synthesis 1 (Synthesis of Intermediate 1)

Under an argon gas flow, 10 g of p-terphenyl, 12 g of iodine, 4.9 g of periodic acid dihydrate, 20 ml of water, 170 ml of acetic acid and 22 ml of sulfuric acid were placed into a 300-ml three-necked flask. After stirring the resultant solution at 65°C for 30 min, the reaction was allowed to proceed at 90°C for 6 h. The resultant mixture was poured into iced water, and then filtered. After washed with water, the filtrate was further washed with methanol, to obtain 67 g of white powder, which was analyzed by FD-MS and identified as Intermediate 1 from the main peak of m/z =482 attributable to C₁₈H₁₂I₂ =482.

### Synthesis 2 (Synthesis of Intermediate 2)

Under an argon gas flow, 11.1 g of 1-acetamidenaphthalene (available from TOKYO CHEMICAL INDUSTRY CO., LTD.), 15.4 g of 4-bromobiphenyl (available from TOKYO CHEMICAL INDUSTRY CO., LTD.), 1.14 g of copper (I) iodide (available from HIROSIMA WAKO CO., LTD.), 1.06 g of N,N'-dimethylethylenediamine (available from ALDRCH CO., LTD), 20.0 g of potassium carbonate (available from HIROSIMA WAKO CO., LTD.), and 100 ml of xylene were placed into a 300-ml three-necked flask, and the reaction was allowed to proceed at 130°C for 36 h.
The resultant solution was cooled down, filtered and washed with toluene. Further, after washing with water and methanol, the solution was dried, to obtain 15.0 g of pale yellow powder.
Into a 300-ml three-necked flask, 15.0 g of the above powder, 17.6 g of potassium hydroxide (available from HIROSINIA WAKO CO., LTD.), 15 ml of ion-exchanged water, 20 ml of xylene (available from HIROSIMA WAKO CO., LTD.) and 10 ml of EtOH (available from HIROSIMA WAKO CO., LTD.) were placed and the resultant solution was refluxed for 36 h. After the reaction was completed, extraction with toluene was carried out and the extract was dried over magnesium sulfate. The dried substance was condensed under reduced pressure and the crude product was purified through a column. The purified product was re-crystallized from toluene and the crystal collected by filtration was dried, to obtain 11.2 g of white powder, which was analyzed by FD-MS and identified as Intermediate 2 from the main peak of m/z = 295 attributable to C₂₂H₁₇N = 295.

### Synthesis 3 (Synthesis of Intermediate 3)

Reaction was conducted in the same manner as Synthesis 2 except that 25.6 g of 4-bromo-p-terphenyl was used instead of 15.4 g of 4-bromobiphenyl, to obtain 11.3 g of white powder, which was analyzed by FD-MS and identified as Intermediate 3 from the main peak of m/z = 482 attributable to C₂₈H₂₁N = 371.

The aromatic amine derivatives of the present invention having the following structural formulae were produced in Synthesis Examples 1 to 3.

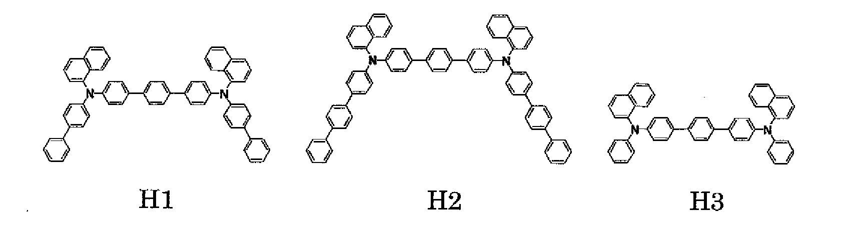

### Synthesis Example 1 (Synthesis of Compound H1)

Under an argon gas flow, 5.8 g of Intermediate 1, 5.9 g of Intermediate 2, 2.7 g of t-butoxy sodium (available from HIROSIMA WAKO CO., LTD.), 186 mg of tris(dibenzylideneacetone) dipalladium(0) (available from ALDRICH CO., LTD), 82 mg of tri-t-butylphosphine and 200 mL of dehydrated toluene were placed and the reaction was allowed to proceed at 80°C for 8 h.
The resultant solution was cooled down, added with 500 ml of water and filtered through sellite. The resultant filtrate was extracted with toluene, and the extract was dried over dehydrated magnesium sulfate. The dried product was condensed under reduced pressure and the crude product was purified through a column. The purified product was re-crystallized from toluene and the crystal collected by filtration was dried , to obtain 4.6 g of pale yellow powder, which was analyzed by FD-MS (Field Desorption Mass Spectrum) and identified as Compound H1 from the main peak of m/z =817 attributable to C₆₂H₄₄N₂ =817.

### Synthesis Example 2 (Synthesis of Compound H2)

Reaction was conducted in the same manner as Synthesis Example 1 except that 7.4 g of Intermediate 3 was used instead of Intermediate 2, to obtain 5.3 g of pale yellow powder, which was analyzed by FD-MS and identified as Compound H2 from the main peak of m/z = 969 attributable to C₇₄H₅₂N₂ = 969.

### Synthesis Example 3 (Synthesis of Compound H3)

Reaction was conducted in the same manner as Synthesis Example 1 except that 4.2 g of N-phenyl-1-naphthylamine was used instead of Intermediate 2, to obtain 3.1 g of pale yellow powder, which was analyzed by FD-MS and identified as Compound H3 from the main peak of m/z = 664 attributable to C₅₀H₃₆N₂ = 664.

### Example 1 (Fabrication of organic EL device)

A glass substrate (product of GEOMATEC Company) of 25 mm ×75 mm × 1.1 mm thickness having an ITO transparent electrode was cleaned by application of ultrasonic wave in isopropyl alcohol for 5 min and then by exposure to ozone generated by ultraviolet light for 30 min.
The cleaned glass substrate having the transparent electrode lines was attached to a substrate holder of a vacuum vapor deposition apparatus. On the surface of the cleaned substrate at the side having the transparent electrode, a film of Compound H232 below having a thickness of 60 nm was formed so as to cover the transparent electrode. The formed film of H232 worked as the electron hole injecting layer. A layer of the Compound H1 as a hole transporting material having a thickness of 20 nm was formed over the film of H232. The formed film worked as the hole transporting layer. Further, Compound EM1 below was deposited thereby forming a film having a thickness of 40 nm. At the same time, the following amine compound D1 having styryl group as a light emitting molecule was deposited in a weight ratio of EM1:D1 = 40:2. The formed film worked as a light emitting layer.
On the film formed above, a film of Alq having a thickness 10 nm was formed. The formed film worked as an electron injecting layer. Thereafter, Li (lithium source: product of SAES GETTERS Company) as a reductive dopant and Alq were binary vapor deposited to form an Alq:Li film (film thickness: 10 nm) as the electron injecting layer (or the cathode). On the Alq:Li film, metallic aluminum was vapor deposited to form a metal cathode, thereby fabricating an organic EL device.
The resultant organic EL device was measured for the emission efficiency and observed for the luminescent color. The emission efficiency at 0 mA/cm² was calculated from the luminance measured by CS1000 (Trade name, product by MINOLTA). The half lifetime of emission when driven by constant DC current at an initial luminance of 5000 cd/m² and room temperature was measured. The results are shown in Table 1.

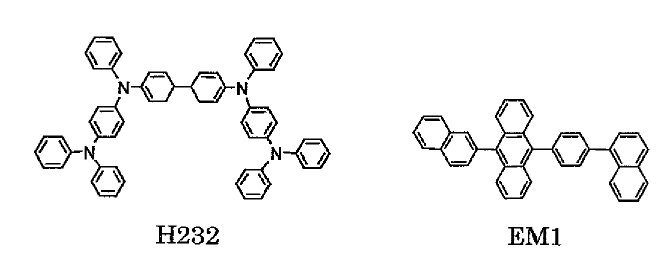

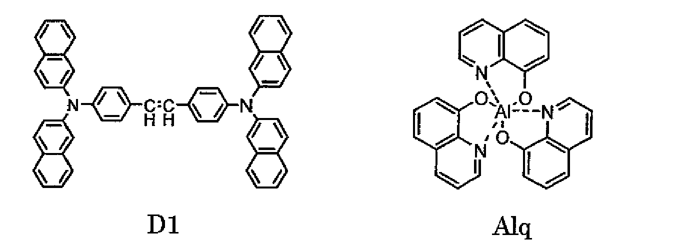

### Examples 2 to 3 (Fabrication of organic EL devices)

Organic EL devices were fabricated in the same manner as in Example 1 except that compounds described in Table 1 were used as the hole transporting material instead of Compound H1.
Each of the organic EL devices was measured for the emission efficiency and observed from the luminescent color. The half lifetime of emission when driven by constant DC current at an initial luminance of 5000 cd/m² and room temperature was measured. The results are shown in Table 1.

### Comparative Examples 1 to 3

Organic EL devices were fabricated in the same manner as in Example 1 except that Comparative Compounds 1 to 3 below were used as the hole transporting material instead of Compound H1.
Each of the organic EL devices was measured for the emission efficiency and observed from the luminescent color. The half lifetime of emission when driven by constant DC current at an initial luminance of 5000 cd/m² and room temperature was measured. The results are shown in Table 1.

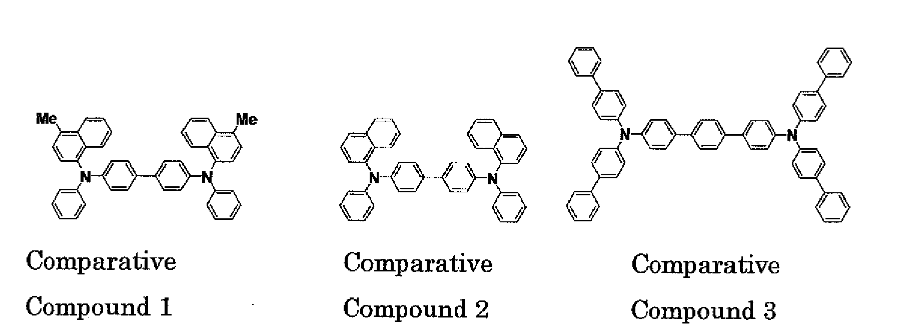

**Table 1**

| Hole transporting material | | Emission efficiency (cd/A) | luminescent color | Half lifetime (h) |
|---|---|---|---|---|
| Examples | | | | |
| 1 | H1 | 6.7 | Blue | 410 |
| 2 | H2 | 6.8 | Blue | 360 |
| 3 | H3 | 6.3 | Blue | 370 |
| 4 | H1 | 6.6 | Blue | 400 |

| Comparative Examples | | | | |
|---|---|---|---|---|
| 1 | Comparative Compound 1 | 4.7 | Blue | 110 |
| 2 | Comparative Compound 2 | 4.9 | Blue | 280 |
| 3 | Comparative Compound 3 | 6.3 | Blue | 240 |
| 4 | Comparative Compound 1 | 4.8 | Blue | 120 |

### Example 4 (Fabrication of organic EL device)

An organic EL device was fabricated in the same manner as in Example 1 except that arylamine compound D2 below was used instead of the amine compound D1 having styryl group. In the following compounds, Me represents a methyl group.
The organic EL device was measured for the emission efficiency and observed from the luminescent color. The half lifetime of emission when driven by constant DC current at an initial luminance of 5000 cd/m² and room temperature was measured. The results are shown in Table 1.

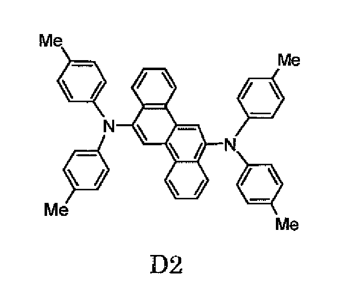

### Comparative Example 4

Organic EL device was fabricated in the same manner as in Example 1 except that the Comparative Compound 1 was used as the hole transporting material instead of Compound H1.
The organic EL device was measured for the emission efficiency and observed from the luminescent color. The half lifetime of emission when driven by constant DC current at an initial luminance of 5000 cd/m² and room temperature was measured. The results are shown in Table 1.

### INDUSTRIAL APPLICABILITY

As described in detail above, the aromatic amine derivative of the present invention suppresses crystallization because the intermolecular interaction is small because of its steric hindrance. Therefore, the yield in the fabrication of organic EL devices is improved, and EL devices with a prolonged lifetime and a high emission efficiency are obtained. Particularly, by combining a blue emission device, the lifetime of EL devices is remarkably prolonged.

## Claims

1. An aromatic amine derivative represented by the following general formula (1). 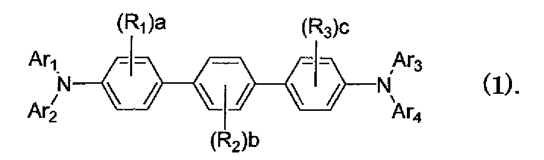 wherein Ar₁ to Ar₄ each independently represents a substituted or unsubstituted aryl group having 5 to 50 ring atoms and at least one of Ar₁ to Ar₄ represents a substituted or unsubstituted aromatic fused ring group having 5 to 50 ring atoms;
R₁ to R₃ each independently represents a hydrogen atom, a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen atom, a cyano group, a nitro group, a hydroxy group or a carboxy group;
a, b and c each independently represents an integer of 0 to 4;
with the proviso that when any of a, b and c is an integer of 2 or greater, plural groups of corresponding R₁ to R₃ may be bonded to each other to form a saturated or unsaturated, five- or six-membered ring structure which may be substituted.

2. The aromatic amine derivative according to claim 1, wherein both Ar₁ and Ar₃ in the general formula (1) are substituted or unsubstituted aromatic fused ring groups having 5 to 50 ring atoms.

3. The aromatic amine derivative according to claim 1 or 2, wherein both Ar₁ and Ar₃ in the general formula (1) are α-naphthyl groups or β-naphthyl groups.

4. The aromatic amine derivative according to any one of claims 1 to 3, wherein Ar₂ and Ar₄ in the general formula (1) are the same as each other.

5. The aromatic amine derivative according to any one of claims 1 to 3, wherein both Ar₂ and Ar₄ in the general formula (1) are phenyl groups.

6. The aromatic amine derivative according to any one of claims 1 to 3, wherein both Ar₂ and Ar₄ in the general formula (1) are biphenylyl groups.

7. The aromatic amine derivative according to any one of claims 1 to 3, wherein both Ar₂ and Ar₄ in the general formula (1) are terphenyl groups.

8. The aromatic amine derivative according to any one of claims 1 to 7, wherein the aromatic amine derivative is used as a material for organic electroluminescence devices.

9. The aromatic amine derivative according to any one of claims 1 to 7, wherein the aromatic amine derivative is used as a hole transporting material for organic electroluminescence devices.

10. An organic electroluminescence device which comprises one or more organic thin film layers including at least one light emitting layer sandwiched between a cathode and an anode, wherein at least one of the organic thin film layers comprises the aromatic amine derivative according to any one of claims 1 to 7 singly or as its mixture component.

11. The organic electroluminescence device according to claim 10, wherein the organic thin film layers include a hole transporting layer, and the hole transporting layer comprises the aromatic amine derivative.

12. The organic electroluminescence device according to claim 11, wherein the hole transporting layer comprises the aromatic amine derivative as its essential component.

13. The organic electroluminescence device according to claim 11, wherein the light emitting layer comprises at least one of a styrylamine compound and an arylamine compound.

14. The organic electroluminescence device according to any one of claims 10 to 13, which emits blue light.
